# EUROPÄISCHE PATENTSCHRIFT

(11) **EP 1 210 309 B1**
(45) Veröffentlichungstag und Bekanntmachung des Hinweises auf die Patenterteilung: **03.12.2003**
(21) Anmeldenummer: 00964071.5
(22) Anmeldetag: 31.08.2000
(51) Int. Cl.: C07C 29/152, C07C 29/151, B01J 8/02

(54) **VERFAHREN UND ANLAGE ZUR METHANOLHERSTELLUNG**
METHOD AND ARRANGEMENT FOR PRODUCING METHANOL
PROCEDE ET INSTALLATION POUR LA PRODUCTION DE METHANOL

(30) Priorität: 07.09.1999 DE 19942736; 08.10.1999 DE 19948585
(43) Veröffentlichungstag der Anmeldung: 05.06.2002
(73) Patentinhaber: Uhde GmbH, 44141 Dortmund (DE)
(72) Erfinder: BÄHNISCH, Hans-Joachim, 44227 Dortmund (DE)
(74) Vertreter: Patentanwälte Meinke, Dabringhaus und Partner
(86) Internationale Anmeldenummer: EP0008486
(87) Internationale Veröffentlichungsnummer: WO01017936

(56) Entgegenhaltungen:
- WO-A-97/31707

## Beschreibung

Die Erfindung bezieht sich auf ein Verfahren zur Methanolsynthese aus Wasserstoff, Kohlenmonoxid und Kohlendioxid unter Druck mit wenigstens einer Synthesestufe der im Oberbegriff des Anspruches 1 angegebenen Gattung.

Es sind eine Reihe von Anlagen bzw. Verfahren zur katalytischen Methanolsynthese bekannt, wobei für die Fülle der Lösungen hier als Beispiel die DE-21 17 060, DE-25 29 591, DE-32 20 995, DE-35 18 362, US-2 904 575 und die DE-41 00 632 genannt seien.

Aus der WO 97/31707 ist ein Verfahren bekannt, bei dem ein Trimmerhitzer vor einem entsprechenden Gas/Gas-Wärmetauscher angeordnet ist, wodurch die Temperaturdifferenz des letzteren verkleinert und somit dessen Fläche vergrößert wird. Eine derartige Flächenvergrößerung soll durch die vorliegende Erfindung verhindert werden. In der WO 97/31707 ist auch eine Technologie beschrieben, bei der der Wärmetauscher in dem Reaktor integriert ist, was zwingend einen zusätzlichen Wärmetauscher notwendig macht, weil die Reaktionsbedingungen sonst instabil sind.

In der DE-40 28 750 der Anmelderin wird ein Ofen (dort 6) zur Erreichung der notwendigen Reaktionstemperatur am Eintritt des adiabaten Reaktors und als indirekter Aufheizer für einen Aufkocher (dort 11) in der Destillation eingesetzt. Dort wird nur ein kleiner Teil der Reaktionswärme des Reaktors für die Aufheizung der Reaktionseintrittsgase verwendet. Der Aufkocher dient dort nur dazu, die nachfolgende Destillation anzutreiben.

In der DE-40 04 862 der Anmelderin wird ein zusätzlicher Wärmetauscher beschrieben, der allerdings dort nur als Anfahrwärmetauscher eingesetzt wird, dessen Verwendung jedoch keine Verringerung der übrigen Wärmetauscherflächen zur Folge hat.

Wie in der DE-41 00 632 beschrieben, ist es vorteilhaft, mehrere Synthesestufen mit Reaktoren einzusetzen, deren erzeugtes Methanol auszukondensieren, vom Synthesegas abzuscheiden und nach jedem einzelnen Reaktor als separaten Methanol-Produktstrom abzuziehen. Bei der Abkühlung des die Reaktoren verlassenden Methanol-Synthesegas-Gemisches ist es wirtschaftlich, die abzugebende Wärme wenigstens zum Teil dazu zu nutzen, die Eintrittsströme in die jeweiligen Reaktoren mittels Gas/Gas-Wärmetauschern zuvor aufzuwärmen.

Als nachteilig stellte sich im langjährigen Betrieb jedoch heraus, daß sich das üblicherweise in den Methanolreaktoren eingesetzte Katalysatormaterial in seiner Aktivität im Laufe der Zeit derart verändert, daß immer höhere Reaktortemperaturen erforderlich werden, um eine optimale Ausbeute zu erreichen und wobei allerdings in den Zonen nach den Gaseintrittsbereichen der Reaktoren solche höheren Reaktortemperaturen nach einiger Zeit nicht mehr erreichbar sind. Aufgrund des verringerten Reaktionsumsatzes wird sowohl weniger Methanol als auch weniger Dampf produziert, der bei der Kühlung der Methanolreaktoren üblicherweise als Koppelprodukt anfällt.

Aufgabe der Erfindung ist es, die genannten Nachteile zu überwinden und insbesondere die Möglichkeit zu schaffen, Wärmetauscherfläche einzusparen und/oder die Ausbeute unter besonderer Berücksichtigung der veränderlichen Katalysatoraktivität zu erhöhen.

Mit einem Verfahren der eingangs bezeichneten Art wird diese Aufgabe gemäß der Erfindung durch die kennzeichnenden Merkmale des Anspruches 1 gelöst. Dabei ist vorgesehen, daß in zumindest einer Synthesestufe das für den Einsatz in einem katalysatorgefüllten Methanolreaktor bestimmte Gasgemisch direkt vor Zugabe in besagten Mechanolreaktor in einem zusätzlichen Trimmerhitzer aufgeheizt wird.

Ausgestaltungen der Erfindung bestehen u.a. darin, daß
- die Temperatur des für den Einsatz in den katalysatorgefüllten Reaktoren bestimmten Gasgemisches nach seiner Erhitzung durch den zusätzlichen Trimmerhitzer gemessen wird,
- die Wärmezufuhr des zusätzlichen Trimmerhitzers geregelt werden kann, und
- der zusätzliche Trimmerhitzer mit einem Fremdmedium, z.B. Dampf, beheizt wird.

Der Hauptvorteil einer solchen Vorgehensweise liegt darin, daß der Gas/Gas-Wärmetauscher, der den austretenden Strom aus dem Reaktor gegen den eintretenden Strom abkühlt, deutlich kleiner dimensioniert werden kann. Überraschenderweise zeigt sich auch, daß bei nachlassender Katalysatoraktivität durch Nachregelung der Reaktoreintrittstemperatur stets eine optimale Ausbeute erreicht werden kann.

Da ein beispielsweise dampfbeheizter Trimmerhitzer vielfach bessere Wärmeübertragungseigenschaften als ein Gas/Gas-Wärmetauscher besitzt und weil auch die erforderliche Sicherheitsreserve allein dem Trimmerhitzer zugerechnet werden kann, ergibt sich "unter dem Strich" sogar eine Einsparung an Investitionskosten. Außerdem zeigt sich, daß die im Trimmerhitzer über die Katalysatorlebensdauer einzusetzende Dampfmenge kleiner ist als der Verlust an Koppelprodukt für den Fall von Einbußen aufgrund verringerten Reaktionsumsatzes. Somit erhöht sich trotz des zusätzlichen Dampfbedarfs unter dem Strich die erzeugte Menge an Dampf. Außerdem zeigt sich, daß sich der Inbetriebnahmezeitraum einer solchen Anlage erheblich verkürzen läßt, wenn Trimmerhitzer als Starterhitzer verwendet werden können.

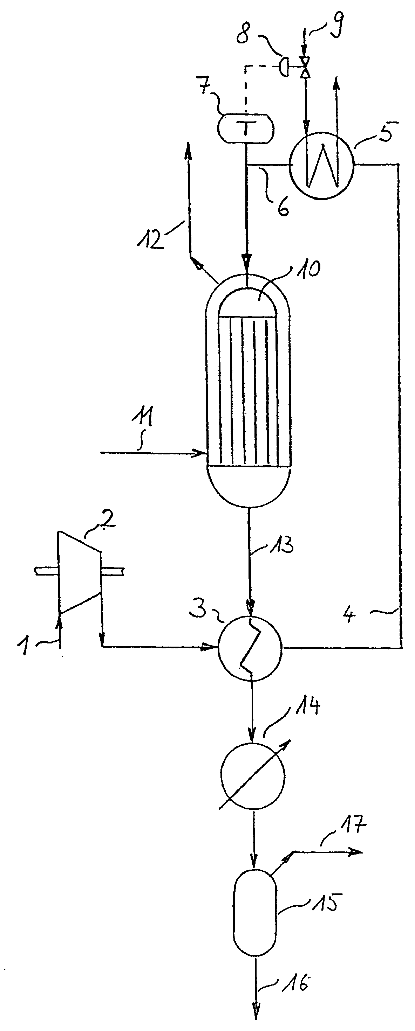

Weitere Vorteile, Einzelheiten und Merkmale der Erfindung ergeben sich aufgrund der nachfolgenden Beschreibung sowie anhand der Zeichnung. Diese zeigt in vereinfachter Darstellung eine von ansonsten mehreren, in der Regel identisch aufgebauten Stufen einer Methanolsyntheseanlage.

Zur Verdichtung des mit 1 bezeichneten Einsatzgases ist ein Verdichter 2 vorgesehen, wobei im weiteren Gasweg ein Gas/Gas-Wärmetauscher 3 zur Vorwärmung des Einsatzgases vorgesehen ist. Das so vorgewärmte Gas wird über die Leitung 4 einem Trimmerhitzer 5 zugeführt, der das Einsatzgas 1 auf die gewünschte Temperatur erhitzt. Zur Messung der Temperatur ist in der Zuführleitung 6 eine Temperaturmeß- und - regeleinrichtung 7 vorgesehen, wobei mittels eines Dampfregelventiles 8 die Dampfzufuhr 9 so geregelt wird, daß stets eine optimale Gaseintrittstemperatur im katalysatorgefüllten Methanolreaktor 10 eingestellt werden kann.

Im katalysatorgefüllten Methanolreaktor 10 reagiert das Einsatzgas unter Wärmeentwicklung. Die Reaktionswärme wird genutzt, um Dampf 12 aus Speisewasser 11 zu erzeugen. Das heiße Produktgas 13 gibt einen Teil seiner Wärmeenergie im Gas/Gas-Wärmetauscher 3 ab. Das im Reaktor erzeugte, gasförmige Methanol wird im Methanolkondensator 14 aus dem Synthesegas auskondensiert, im Kondensatabscheider 15 vom Synthesegas abgetrennt und als Produkt-Methanol 16 abgeführt. Das verbleibende Synthesegas 17 kann in der nachfolgenden Stufe weiterverwendet werden.

In der erfindungsgemäßen Anlage erfolgt die Regelung der Reaktion im Methanolreaktor 10 hauptsächlich über eine in der Figur nicht näher dargestellte Druckregelung für das Kühlmedium (Wasser 11 bzw. Dampf 12), welches die katalysatorgefüllten Rohre umgibt. Sinkt die Katalysatoraktivität, ist es erforderlich, das gesamte Temperaturniveau im Reaktor anzuheben, was durch Hochregelung des Druckes des Kühlmediums erfolgt, wodurch die Verdampfungstemperaur des Kühlmediums steigt. Dadurch erhöht sich die Temperatur im Katalysator und die Austrittstemperatur 13 erhöht sich ebenfalls.

Über den Wärmetauscher 3 erhöht sich ebenfalls die Temperatur in der Leitung 4, jedoch noch nicht in dem für das Erreichen optimaler Reaktionsbedingungen erforderlichen Maß. Dies wird durch den Trimmerhitzer 5 erreicht. Erkennbar erfüllt der Trimmerhitzer 5 die wesentliche Aufgabe, den Flächenbdarf des Gas/Gas-Wärmetauschers 3 zu verhindern, zum einen dadurch, daß die Temperaturdifferenz vergrößert wird, zum anderen dadurch, daß auf den üblichen Sicherheitszuschlag verzichtet werden kann, und zwar insbesondere deswegen, weil der Wärmeübergang um einen Faktor sieben im Trimmerhitzer besser ist. Der Zuschlag der Fläche fällt dort daher sehr viel geringer aus, als beim Wärmetauscher, ein wesentlicher Vorteil der vorliegenden Erfindung.

Natürlich ist das beschriebene Ausführungsbeispiel noch in vielfacher Hinsicht abzuändern. So kann beispielsweise der Trimmerhitzer ggf. zweistufig ausgebildet sein u. dgl. mehr.

## Patentansprüche

1. Verfahren zur Methanolsynthese aus Wasserstoff, Kohlenmonoxid und Kohlendioxid unter Druck mit wenigstens einer Synthesestufe, die wenigstens einen katalysatorgefüllten Methanolreaktor enthält, wobei bei mehreren Stufen wenigstens eine Stufe mit einem Verdichter ausgestattet ist und wobei alle Synthesestufen mit eigenem Produktabzug für Methanol und eigenem Gas/Gas-Wärmetauscher zur Übertragung von Wärmeenergie des den Methanolreaktor verlassenden Gasgemisches an das zum Einsatz im Methanolreaktor vorgesehene Gasgemisch ausgestattet sind,
**dadurch gekennzeichnet,**
**daß** in zumindest einer Synthesestufe das für den Einsatz in einem katalysatorgefüllten Methanolreaktor bestimmte Gasgemisch direkt vor Zugabe in besagten Methanolreaktor in einem zusätzlichen Trimmerhitzer aufgeheizt wird.

2. Verfahren nach Anspruch 1,
**dadurch gekennzeichnet,**
**daß** die Temperatur des für den Einsatz in einem katalysatorgefüllten Methanolreaktor bestimmte Gasgemisch nach seiner Erhitzung durch den Trimmerhitzer gemessen wird.

3. Verfahren nach Anspruch 1 und 2,
**dadurch gekennzeichnet,**
**daß** die Wärmezufuhr des Trimmerhitzers geregelt werden kann.

4. Verfahren nach einem der vorangegangenen Ansprüche,
**dadurch gekennzeichnet,**
**daß** der Trimmerhitzer mit einem Fremdmedium, vorzugsweise Dampf, beheizt wird.

## Claims

1. Method for synthesising methanol from hydrogen, carbon monoxide and carbon dioxide under pressure, using at least one synthesis step containing at least one catalyst-filled methanol reactor, where if there are a plurality of steps at least one step is equipped with a compressor and each of the synthesis steps is equipped with its own product take-off point for methanol and its own gas/gas heat exchanger for transferring thermal energy from the gas mix as it leaves the methanol reactor to the gas mix provided for use in the methanol reactor,
**characterised in that** in at least one synthesis step the gas mix intended for use in a catalyst-filled methanol reactor is heated in an additional trimming heater directly before being charged into said methanol reactor.

2. Method according to claim 1,
**characterised in that** the temperature of the gas mix intended for use in a catalyst-filled methanol reactor is measured after it has been heated by the trimming heater.

3. Method according to claims 1 and 2,
**characterised in that** the supply of heat to the trimming heater can be regulated.

4. Method according to any of the preceding claims,
**characterised in that** the trimming heater is heated using an external medium, preferably steam.

## Revendications

1. Procédé pour la synthèse de méthanol à partir d'hydrogène, de monoxyde de carbone et de dioxyde de carbone sous pression avec au moins un étape de synthèse, qui contient au moins un réacteur de méthanol rempli de catalyseur, dans le cas de plusieurs étapes au moins un étape étant équipé d'un condenseur et tous les étages de synthèse étant équipés de leur propre extracteur pour le méthanol et de leur propre échangeur de chaleur gaz-gaz pour transmettre l'énergie thermique du mélange gazeux quittant le réacteur de méthanol au mélange gazeux prévu pour être utilisé dans le réacteur de méthanol, **caractérisé en ce que**, à au moins un étape de synthèse, le mélange gazeux destiné à être utilisé dans un réacteur de méthanol rempli de catalyseur est réchauffé dans un réchauffeur d'appoint supplémentaire directement avant ajout dans ledit réacteur de méthanol.

2. Procédé selon la revendication 1, **caractérisé en ce que** la température du mélange gazeux destiné à être utilisé dans un réacteur de méthanol rempli de catalyseur est mesurée après son réchauffement par le réchauffeur d'appoint.

3. Procédé selon la revendication 1 et 2, **caractérisé en ce que** l'arrivée de chaleur du réchauffeur d'appoint peut être réglée.

4. Procédé selon l'une des revendications précédentes, **caractérisé en ce que** le réchauffeur d'appoint est réchauffé avec un milieu extérieur, de préférence de la vapeur.
